# EUROPEAN PATENT APPLICATION

(11) **EP 4 458 408 A1**
(43) Date of publication of application: **06.11.2024**
(21) Application number: 22914914.1
(22) Date of filing: 28.12.2022
(51) Int. Cl.: A61N 5/10

(54) **CALIBRATION METHOD, CALIBRATION DEVICE AND CALIBRATION SYSTEM OF LASER POSITIONING SYSTEM**

(30) Priority: 30.12.2021 CN 202111660492
(71) Applicant: Neuboron Therapy System Ltd., Xiamen, Fujian 361026 (CN)
(72) Inventor: SHU, Di-Yun, Nanjing, Jiangsu 211112 (CN); GONG, Qiu-Ping, Nanjing, Jiangsu 211112 (CN); LIU, Xing-Yan, Nanjing, Jiangsu 211112 (CN)
(74) Representative: Gunzelmann, Rainer
(86) International application number: PCT/CN2022/142810
(87) International publication number: WO 2023/125662

(57) **Abstract**

The present application provides a calibration method, calibration device and calibration system of a laser positioning system. The calibration method comprises: arranging calibration devices in a first chamber and a second chamber, respectively, each calibration device comprising a calibration phantom, and the relative position between the calibration phantom of the first chamber and a preset center point of the first chamber being the same as the relative position between the calibration phantom of the second chamber and a preset center point of the second chamber; and comparing the position of a first positioning mark on the calibration phantom of the first chamber and the position of a second positioning mark on the calibration phantom of the second chamber. The technical solution provided by the present application can avoid the problem of deviation of an irradiation position to a patient from a correct treatment position due to inconsistency of a first laser positioning system of the first chamber and a second laser positioning system of the second chamber, and reduce or avoid unnecessary radiation dose to the patient.

## Description

### TECHNICAL FIELD

The present application relates to the field of medical technology and, specifically, to a calibration method, calibration device and calibration system of a laser positioning system.

### BACKGROUND

With the development of atomic science, radiation therapy, such as cobalt 60, linear accelerator, and electron beam, has become one of the main means of cancer treatment. However, conventional photon or electron therapy is limited by the physical conditions of the radiation itself, which kills tumor cells while causing damage to a large number of normal tissues in the path of the beam.

In order to reduce the radiation damage to the normal tissues surrounding the tumor, the concept of targeted therapy in chemotherapy has been applied to radiation therapy; For tumor cells with high resistance to radiation, radiation sources with high relative biological effectiveness (RBE), such as proton therapy, heavy particle therapy, neutron capture therapy, etc., are also being actively developed. Among them, neutron capture therapy is a combination of the above two concepts, such as boron neutron capture therapy, which provides a better cancer treatment option than conventional radiation by the specific concentration of boron-containing drugs in the tumor cells, together with precise neutron beam irradiation.

Radiation therapy systems typically include multiple activity spaces, including imaging chamber, irradiation chamber, preparation chamber, etc. The irradiation chamber is a room used to irradiate a neutron beam to a patient for treatment, the irradiation chamber is provided with a laser transmitter to emit a laser beam to the patient for treatment positioning; The preparation chamber is a room designed to perform the preparations required before irradiating a patient with a neutron beam, and is equipped with a laser emitter to emit a laser beam to the patient for simulation positioning.

The layout and structural setup related to positioning in the irradiation chamber and those in the preparation chamber are basically similar, and both have laser positioning systems, collimators and treatment beds.

Where the laser positioning system typically includes a plurality of laser emitters fixedly mounted on the walls and ceiling of the preparation chamber/irradiation chamber, i.e., the walls of both the irradiation chamber and the preparation chamber have laser emitters in exactly the same relative positions. Ensure that the patient's tumor target area is centered on the isocenter of the simulator (in the preparation chamber) or the radiation therapy machine (in the irradiation chamber) by setting up positioning marks in the preparation chamber and irradiation chamber via many irradiations to the patient.

However, the laser positioning system may be offset by factors such as environmental, artificial or temperature difference etc. during use, resulting in inconsistency of the isocenter positions indicated by the laser positioning system in the preparation chamber and the irradiation chamber, thereby causing the irradiation position of the radiation therapy machine on the patient to deviate from the correct treatment position. Positioning the patient for treatment under these conditions is equivalent to the patient creating postural movement during treatment. In this case, the patient is treated less effectively and receives a higher dose of unnecessary radiation compared to the correct treatment position. Therefore, it is important to calibrate the accuracy of the laser positioning system on a regular basis.

### SUMMARY

In view of the above, the present application provides a calibration method, calibration device and calibration system of a laser positioning system to solve a problem in the prior art in which inconsistency of the isocenter positions indicated by the laser positioning systems of the first chamber and second chamber results in the irradiation position of the radiation therapy machine on the patient deviating from the correct treatment position.

In order to achieve the above purposes, the present application provides a method of calibrating a laser positioning system, the method includes:
providing a calibration device in each of a first chamber and a second chamber; where the calibration device includes a calibration die, and a relative position of the calibration die of the first chamber to a predetermined center point of the first chamber is identical with a relative position of the calibration die of the second chamber to a predetermined center point of the second chamber; and
comparing a position of a first positioning mark on the calibration die of the first chamber and a position of a second positioning mark on the calibration die of the second chamber; where the first positioning mark is a positioning mark formed on the calibration die by a laser emitted by a first laser positioning system of the first chamber and the second positioning mark being a positioning mark formed on the calibration die by a laser emitted by a second laser positioning system of the second chamber.

Optionally, the method further includes:
adjusting the first laser positioning system and/or the second laser positioning system based on a deviation if there is a deviation between the position of the first positioning mark on the calibration die and/or the position of the second positioning mark on the standard die.

Optionally, the calibration device further includes a support frame.

Providing the calibration device includes: mounting the support frame, and providing the calibration die at a predetermined position on the support frame.

Optionally, the support frame includes a mounting bracket and a support plate mounted on the mounting bracket for supporting the calibration die.
mounting the support frame includes: mounting the mounting bracket in a predetermined position in the second chamber or the first chamber and adjusts angle and/or height of the support plate relative to the mounting bracket.

Optionally, the support plate is provided with a first scale in a first direction and a second scale in a second direction perpendicular to the first direction; and

Providing the calibration die at the predetermined position on the support frame includes: placing the calibration die at a predetermined scale position on the support plate according to the first scale and the second scale.

Optionally, in each of the first chamber and the second chamber, the support frame is mounted and adjusted to a horizontal level, and the calibration die located in each of the first chamber and the second chamber is placed in the predetermined scale position.

Optionally, the calibration module is provided with a reference mark.

The present application, on the other hand, provides a calibration device for calibrating a laser positioning system, the calibration device is mounted in a first chamber having a first laser positioning system and/or a second chamber having a second laser positioning system.

The calibration device including a calibration die.

A laser emitted by the first laser positioning system forms a first positioning mark on the calibration die when the calibration device is mounted in the first chamber, and a laser light emitted by the second laser positioning system forms a second positioning mark on the calibration die when the calibration device being mounted in the second chamber; consistency of the first laser positioning system and the second laser positioning system is determined based on a position of the first positioning mark on the calibration die and a position of the second positioning mark on the calibration die.

Optionally, the calibration device further includes a support frame, the support frame is mounted in the first chamber or the second chamber, and the calibration die is provided on the support frame.

Optionally, the support frame includes a mounting bracket for mounting and a support plate provided on the mounting bracket, the calibration die is selectively provided on the support plate.

The support plate is adjustable in angle and/or height relative to the mounting bracket.

The support plate is provided with a first scale in a first direction and a second scale in a second direction perpendicular to the first direction, and according to the first scale and the second scale, the calibration die is provided at a predetermined scale position on the support plate.

Optionally, the mounting bracket is provided with an adjusting structure located below the support plate, the support plate is provided with a positioning structure on a lower surface of the support plate which cooperates with the adjusting structure, the angle and/or height of the support plate relative to the mounting bracket are adjusted by adjusting the adjusting structure. Where the adjusting structure is optionally provided as an adjusting bolt and the positioning structure is correspondingly provided as a positioning hole.

Optionally, the calibration module is provided with a reference mark.

According to another aspect of this application, it also provides a calibration system, the calibration system includes a first chamber provided with a first laser positioning system, a second chamber provided with a second laser positioning system, and a calibration device above described.

The calibration device is provided in the first chamber and the second chamber, respectively; when a relative position between the calibration die of the first chamber and a predetermined center point of the first chamber and a relative position between the calibration die of the second chamber and a predetermined center point of the second chamber is identical, a position of a first positioning mark on the calibration die is compared with a second positioning mark of the second positioning mark on the calibration die to determine consistency of the first laser positioning system and the second laser positioning system; where the first positioning mark is a positioning mark formed on the calibration die of the first chamber by a laser emitted by the first laser positioning system and the second positioning mark is a positioning mark formed on the calibration die of the second chamber by a laser emitted by the second laser positioning system.

Optionally, each of the first chamber and the second chamber is provided with a collimator and a collimator mounting structure for mounting the collimator, and the support frame of the calibration device is mounted on the collimator mounting structure.

A predetermined center point of each of the first chamber and the second chamber corresponds to the collimator in position relationship, and is exactly consistent in spatial dimension.

In the technical solution provided by the present application, by comparing the first positioning mark formed on the calibration die by the first laser positioning system in the first chamber and the second positioning mark formed on the calibration die by the second laser positioning system in the second chamber, it can be determined whether there is any deviation between the first laser positioning system and the second laser positioning system, thereby avoiding that the irradiation position of the radiation therapy machine of the second chamber deviates from the correct treatment position, which brings ineffective treatment and unnecessary radiation dose to patient.

Other features and advantages of the present application will be described in detail in the subsequent specific embodiments section.

### BRIEF DESCRIPTION OF THE DRAWINGS

The accompanying drawings, which form part of this application, are used to provide further understanding of the present application, and the schematic embodiments of the application and the description thereof are used to explain the present application and do not constitute an undue limitation of the present application. In the accompanying drawings:
FIG. 1 shows a schematic structural diagram of a calibration device provided in a first chamber and a second chamber, respectively, according to an embodiment of the present application;
FIG. 2 shows a schematic structural diagram of an embodiment in which a first collimator and a first laser positioning system are provided in a first chamber;
FIG. 3 shows a schematic structural diagram of an embodiment in which a second collimator and a second laser positioning system are provided in a second chamber;
FIG. 4 shows a schematic diagram of the structure of a calibration device according to one embodiment of the present disclosure;
FIG. 5 shows a schematic view of the structure of the calibration device shown in FIG. 4 as viewed from one side;
Figure 6 shows a schematic diagram of the support plate provided with a scale.

### Description of the accompanying symbols

100-first chamber; 101-first collimator; 102-first laser emitter; 200-second chamber; 201-second collimator; 202-second laser emitter; 1-calibration die; 2- support frame; 21-support plate; 22- mounting bracket; 23- support bolt.

### DETAILED DESCRIPTION OF THE EMBODIMENTS

The technical solutions in the embodiments of the present application will be clearly and completely described below in conjunction with the accompanying drawings in the embodiments of the present application, and it is clear that the embodiments described are only a part of the embodiments of the present application, rather than all of the embodiments. Based on the embodiments in the present application, all other embodiments obtained by a person of ordinary skill in the art without making creative labor fall within the scope of protection of the present application. The embodiments and the features in the embodiments in the present application may be combined with each other without conflict.

In the description of the present application, it is to be understood that the terms "center", " portrait", "horizontal", "up ", "down", "left", "right", "vertical", "level", "top", "bottom", "axial", "radial", "circumferential" and the like indicate orientation or positional relationships based on those shown in the accompanying drawings. They are intended only to facilitate and simplify the description of the present application, and do not indicate or imply that the devices or elements referred to must have a particular orientation, be constructed and operated in a particular orientation, and therefore are not to be construed as a limitation of the present application. In addition, "inside and outside" means inside and outside in relation to the contours of the parts themselves.

Furthermore, the terms "first" and "second" are used for descriptive purposes only and are not to be construed as indicating or implying relative importance or implicitly specifying the number of technical features indicated. As a result, a feature defined as "first" or "second" may include at least one such feature, either explicitly or implicitly.

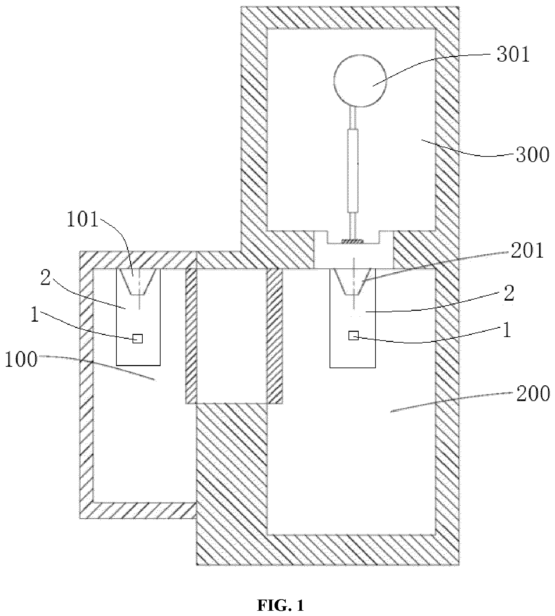

The radiation therapy system includes a first chamber 100 and a second chamber 200. As shown in FIG. 1, the first chamber 100 may be a preparation chamber, a room in which preparation work is performed prior to irradiating a neutron beam to a patient. The preparation work includes fixing the patient to the treatment table, locating the tumor of the patient and making three-dimensional positioning mark, and the like. The second chamber 200 is may be an irradiation chamber, a room for irradiating a neutron beam to a patient for treatment. The preparation chamber 100 is preferably provided on one side of the irradiation chamber 200, with an inlet and outlet for patient access and a door to open or close the inlet and outlet provided therebetween. The charged particle generating chamber 300 generates a charged particle beam through the charged particle generating section 301, and after a series of reactions and decelerations, the charged particle beam forms a neutron beam for treatment and the neutron beam is emitted from the second collimator 201 of the irradiation chamber 200 to the tumor site of the patient.

The first and second chambers include, but are not limited to, preparation or irradiation chambers, but may also be spaces with other functions that need to be posed for alignment during the actual treatment and preparation process.

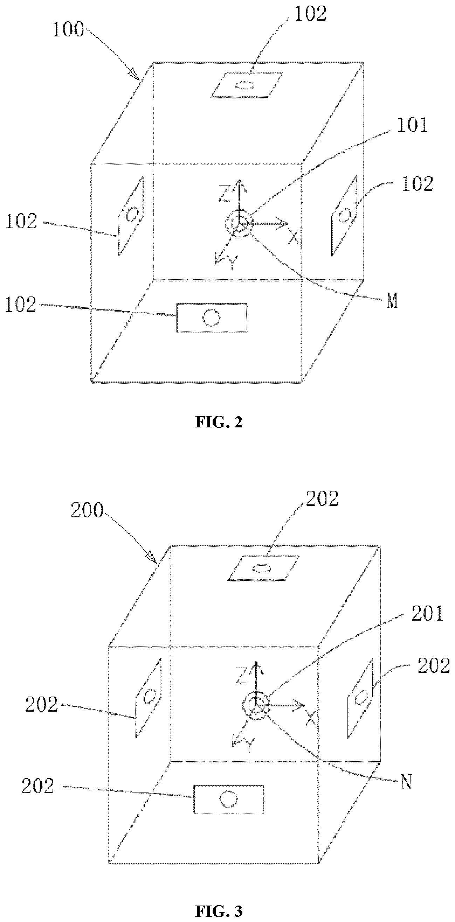

As shown in FIGS. 2 and 3, the preparation chamber 100 is provided with a first laser positioning system, the first laser positioning system including a plurality of first laser emitters 102 disposed on the top wall and each side wall of the preparation chamber 100, respectively, for emitting laser beams to the patient for simulation positioning. The preparation chamber 100 is also provided with a first collimator 101 located on a side wall, this first collimator 101 serves as an analog collimator for positioning reference, and is identical in structure, dimensions and location to the second collimator 201 in the irradiation chamber 200.

The irradiation chamber 200 is provided with a second laser positioning system, the second laser positioning system includes a plurality of second laser emitters 202 provided on the top wall and each side wall of the irradiation chamber 200, respectively. A second collimator 201 for emitting the neutron beam for treatment is also provided on a side wall within the irradiation chamber 200.

In conjunction with the image of the affected area of the patient obtained by using an imaging modality such as CT imaging, after the tumor site is localized and made positioning mark on the patient's body surface by means of the respective first laser emitter 102 of the first laser positioning system in the preparation chamber 100, and then the patient enters into the irradiation chamber 200. In the irradiation chamber 200, the laser light emitted by the respective second laser emitter 202 of the second laser positioning system is aligned and positioned against the predetermined positioning mark on the patient's body surface so that the neutron beam can be aimed at the tumor site for irradiation. The specific marking methods and positioning methods are techniques well known to those of ordinary skill in the art and are not described in detail here.

In an embodiment, the positional relationship between the respective first laser emitter 102 and the first collimator 101 within the preparation chamber 100 needs to be set to be identical in one-to-one correspondence with the positional relationship between the respective second laser emitter 202 and the second collimator 201 within the irradiation chamber 200, respectively. Of course, the preparation chamber 100 may actually be smaller in size than the irradiation chamber 200, and it should be ensured, as far as possible, that the predetermined center point of the preparation chamber 100 and the predetermined center point of irradiation chamber 200 are the same positional relationships in one-to-one correspondence with respect to their respective collimators. That is to say, the absolute spatial dimension of the collimator in relation to the predetermined center point in the preparation chamber 100 and the irradiation chamber 200 should be identical, and be completely uniform in the vertical, level, and anterior-posterior dimensions, so as to enable the neutron beam to be directed to the tumor site for irradiation. Specifically, the center point M of the first collimator 101 is used as a reference point in the preparation chamber 100, and the center point N of the second collimator 201 is used as a reference point in the irradiation chamber 200. Preferably, the coordinate values of the respective first laser emitters 101 need to be identical in one-to-one correspondence with the coordinate values of the respective second laser emitters 201. The intersection point obtained by the intersection of the laser planes emitted by the respective first laser emitters 101 is located on the outlet axis of the first collimator 101, and the intersection point obtained by the intersection of the laser planes emitted by the respective second laser emitter 201 is located on the outlet axis of the second collimator 201, and the absolute positions of the laser intersection point and the center point M of the first laser emitter 101 and the absolute positions of the laser intersection point and the center point N of the second laser emitter 201 are identical.

For this purpose, the first laser positioning system and the second laser positioning system need to be calibrated so that the positional relationship between the two can be the same in one-to-one correspondence, in order to avoid that the treatment position indicated by the second laser positioning system deviates from the correct treatment position in the event of an offset between the two, which may result in the patient not achieving the intended therapeutic effect and being subjected to an unwanted radiation dose.

The present application provides a method of calibrating a laser positioning system, the method including:
As shown in FIG. 1, calibration devices are provided in the preparation chamber100 and the irradiation chamber 200, respectively; where the calibration device includes a calibration die 1, the relative position between the calibration die 1 of the preparation chamber 100 and the predetermined center point of the preparation chamber 200 and the relative position between the calibration die 1 of the irradiation chamber 200 and the predetermined center point of the irradiation chamber 200 are the same; where the predetermined center point of the preparation chamber 200 may be the center point M of the projection of the first collimator 101 on the wall, and correspondingly, the predetermined center point of the irradiation chamber 200 is the center point N of the projection of the second collimator 201 on the wall. Of course, the predetermined center points of the preparation chamber 100 and the irradiation chamber 200 may be other predetermined points corresponding to each other, such as a certain datum point of the treatment table for positioning patient.

Then, the position of the first positioning mark on the calibration die 1 of the preparation chamber 100 and the position of the second positioning mark on the calibration die 1 of the irradiation chamber 200 are compared; where the first positioning mark is a positioning mark formed on the calibration die 1 by a laser emitted by the first laser positioning system of the preparation chamber 100, and the second positioning mark is a positioning mark formed on the calibration die 1 by a laser emitted by the second laser positioning system of the irradiation chamber 200.

Where the first positioning mark and the second positioning mark may be crosshair marks or dots formed by the emitted laser on the surface of the calibration die 1, without limitation here.

Where the calibration device is separately provided in the preparation chamber 100 and the irradiation chamber 200, it may be that after the calibration device is provided in the preparation chamber 100, the position of a first positioning mark is marked by forming a first positioning mark on the calibration die 1, and then the calibration device is placed in the irradiation chamber 200, and the second positioning mark formed in the irradiation chamber is compared with the mark of the first positioning mark, i.e., the calibration device employed by the preparation chamber 100 and the irradiation chamber 200 are the same calibration device. Alternatively, two identical calibration devices are taken in the preparation chamber 100 and the irradiation chamber 200, i.e. a first calibration device is provided in the preparation chamber 100 and a second calibration device is provided in the irradiation chamber 200.

The calibration method of the laser positioning system further includes:
If there is a deviation between the position of the first positioning mark on the calibration die 1 and the position of the second positioning mark on the standard diel, it is indicated that the first laser positioning system of the preparation chamber 100 is not consistent with the second laser positioning system of the irradiation chamber 200. The first laser positioning system and/or the second laser positioning system may be adjusted based on this deviation in relation to the positions of the first positioning mark and the second positioning mark on the calibration die 1. Specifically, the position and angle of the laser projection and the degree of focusing of the laser can be adjusted so that the points of intersection of the laser planes of the first laser positioning system in the preparation chamber 100 and the second laser positioning system in the irradiation chamber 200 are as consistent as possible.

And if the position of the first positioning mark on the calibration die 1 and the position of the second positioning mark on the standard die 1 are identical in one-to-one correspondence and in exactly the same dimensions, respectively, the first laser positioning system of the preparation chamber 100 and the second laser positioning system of the irradiation chamber 200 are consistent and do not need to be adjusted.

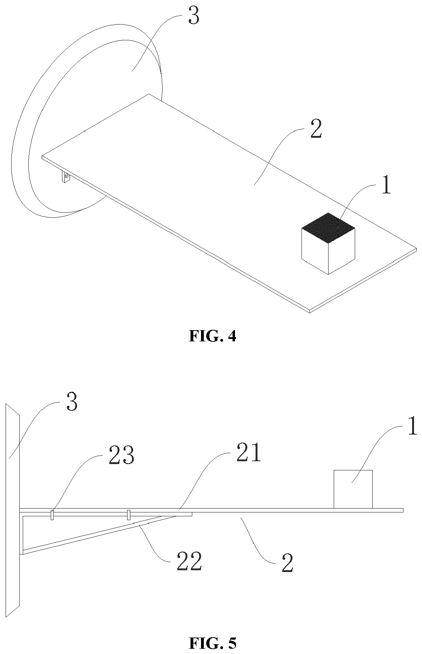

In one embodiment, as shown in Figures 4 and 5, the calibration device further includes a support frame 2. A method of setting up a calibration device includes: installing the support frame 2 and setting the calibration die 1 at the preset position of the support frame 2. Optionally, the support frame 2 can be mounted on the collimator mounting structure, the first collimator 101 in the preparation chamber 100 and the second collimator 201 in the irradiation chamber 200 are both mounted on the wall by means of the collimator mounting structure, and it is possible to mount the support frame 2 on the collimator mounting structure as well (and, of course, may be mounted on other structures), and the connection between the support frame 2 and the collimator mounting structure includes but are not limited to bolt connections, articulation connections, hinge connections, and the like.

Further, the support frame 2 includes a mounting bracket 22 and a support plate 21 mounted on the mounting bracket 22 for supporting the calibration die 1.

The method of installing the support frame 2 includes: mounting the mounting bracket 22 at a predetermined position in the irradiation chamber 200 or the preparation chamber 100, and adjusting angle and/or height of the support plate 21 relative to the mounting bracket 22.

Specifically, the mounting bracket 22 is provided with an angle and/or height adjusting structure, such as an adjusting bolt 23 located below the support plate 21, and the lower surface of the support plate 21 is provided with a positioning hole that cooperates with the adjusting bolt 23. As shown in FIG. 5, the angle and/or height of the support plate 21 relative to the mounting bracket 2 can be adjusted by rotating the adjusting bolt 23, and a level meter can be used to detect the levelness of the support plate 21 during adjustment of the support plate 21 to ensure that the support plate 21 is mounted horizontally, so as to ensure that the calibration die is in a horizontal plane when the calibration die is set.

In this embodiment, the support plate 21 of the support frame 2 is provided with a first scale in a first direction and a second scale in a second direction perpendicular to the first direction.

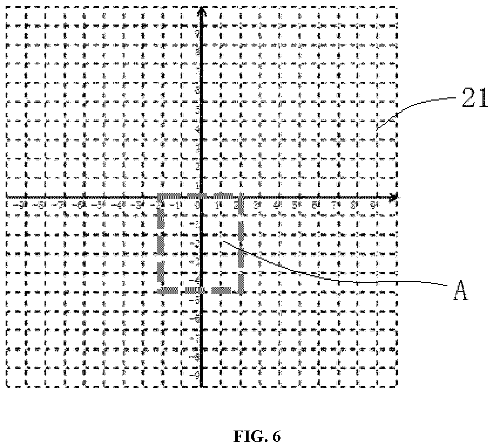

A method of setting the calibration die 1 at a predetermined position of the support frame 2 includes: according to the first scale and the second scale, placing the calibration die 1 at a predetermined scale position on the support plate 21. As shown in FIG. 6, A indicated by the dashed line, is a location for placing the calibration die 1. The location is determined based on the coordinates in the two perpendicular directions, and the calibration die 1 can then be quickly and accurately placed at that location.

When the support plate 21 of the support frame 2 within the irradiation chamber 200 and the preparation chamber 100 are at the same height and in a horizontal position, and the calibration die 1 is at the same scale position on the support plate 21, which can be ensured that the relative positions of the calibration die 1 within the irradiation chamber 200 and the preparation chamber 100 with respect to the predetermined center point are the same, i.e., the coordinate value of the calibration die within the preparation chamber 100 with respect to the predetermined center point and the coordinate value of the calibration die 1 within the irradiation chamber 100 with respect to the predetermined center point are the same.

Preferably, the calibration module 1 is provided with a reference mark, such as a datum line in a plurality of dimensions, a scale, etc., and preferably, the reference mark is aligned with a scale on the support plate. Such a setup is conducive to more accurate and convenient comparison and improved visibility when aligning laser lines in different spaces.

According to another aspect of the present application, there is also provided a calibration device for calibrating a laser positioning system, the calibration device is mounted in a preparation chamber 100 having a first laser positioning system or an irradiation chamber 200 having a second laser positioning system.

The calibration device includes the support frame 2 and the calibration die 1, the support frame 2 is mounted at a predetermined position in the preparation chamber 100 or the irradiation chamber 200, and the calibration die 1 is set on the support frame 2;

The lasers emitted by the respective first laser emitters 101 of the first laser positioning system form a first positioning mark on the calibration die 1 when the calibration device is installed in the preparation chamber 100, and the lasers emitted by the respective second laser emitters 201 of the second laser positioning system form a second positioning mark on the calibration die 1 when the calibration device is installed in the irradiation chamber 200. The consistency of the first laser positioning system and the second laser positioning system is determined based on the position of the first positioning mark on the calibration die and the position of the second positioning mark on the calibration die.

If there is a deviation between the position of the first positioning mark on the calibration die 1 and the position of the second positioning mark on the standard die 1, it is indicated that the first laser positioning system of the preparation chamber 100 is not consistent with the second laser positioning system of the irradiation chamber 200. The first laser positioning system and/or the second laser positioning system may be adjusted based on this deviation in relation to the positions of the first positioning mark and the second positioning mark on the calibration die 1. Specifically, the position, angle, and focus of the laser line projected by each laser module can be adjusted so that the first laser positioning system of the preparation chamber 100 and the second laser positioning system of the irradiation chamber 200 are consistent, which is conducive to improving the accuracy of the subsequent posing. And if the position of the first positioning mark on the calibration die 1 and the position of the second positioning mark on the standard die 1 are identical in one-to-one correspondence and in exactly the same dimensions, respectively, the first laser positioning system of the preparation chamber 100 and the second laser positioning system of the irradiation chamber 200 are consistent and do not need to be adjusted.

In order to facilitate the comparison of the positions of the first positioning mark and the second positioning mark on the calibration die, a scale or a grid may be provided on the calibration die 1, which is conducive to a more rapid and precise alignment verification.

In one embodiment, the support frame 2 includes a mounting bracket 22 for mounting and a support plate 21 provided on the mounting bracket 22, and the calibration die 1 is provided on the support plate 21.

Where the support plate 21 has an angle and/or height adjusting structure relative to the mounting bracket 22.

The support plate 21 is provided with a first scale in a first direction and a second scale in a second direction perpendicular to the first direction, and a placement position of the calibration die 1 on the support plate 21 is determined based on the first scale and the second scale.

Further, the angle and/or height adjusting structure is an adjusting bolt 23 located below the support plate 22, which is provided on the mounting bracket 21. The lower surface of the support plate 21 is provided with a positioning hole that cooperates with the adjusting bolt 23, and the angle and/or height of the support plate 21 relative to the mounting bracket 22 can be adjusted by rotating the adjusting bolt 23.

Another aspect of the present application also provides a calibration system, the calibration system includes a preparation chamber 100 provided with a first laser positioning system, an irradiation chamber 200 provided with a second laser positioning system, and a calibration device as above.

The calibration device is provided in the preparation chamber 100 and the irradiation chamber 200, respectively; and when the relative position between the calibration die 1 of the preparation chamber 100 and the predetermined center point of the preparation chamber 100 and the relative position between the calibration die 1 of the irradiation chamber 200 and the predetermined center point of the irradiation chamber 200 are the same, the position of the first positioning mark on the calibration die 1 of the preparation chamber 100 is compared with the second positioning mark of the second positioning mark on the calibration die 1 of the irradiation chamber 200 to determine the consistency of the first laser positioning system and the second laser positioning system; Where the first positioning mark is a positioning mark formed on the calibration die 1 of the preparation chamber 100 by a laser emitted by the first laser positioning system, and the second positioning mark is a positioning mark formed on the calibration die 1 of the irradiation chamber 200 by a laser emitted by the second laser positioning system.

In one embodiment, the preparation chamber 100 and the irradiation chamber 200 are each provided with a collimator and a collimator mounting structure for mounting the collimator, and the support frame 2 of the calibration device is mounted on the collimator mounting structure. See the above description for the way in which the support frame 2 is attached to the collimator mounting structure and the way in which it is adjusted. Preferably, the first collimator 101 in the preparation chamber 100 and the second collimator 201 in the irradiation chamber 200 may have a consistent quick-loading structure that allows for quick and precise loading of the calibration device, ensuring that the calibration device is in the same position relative to the respective collimator space in both spaces.

Where the predetermined center point is the center point of the projection of the collimator on the wall, i.e., the center point M of the projection of the first collimator 101 on the wall where it is located is selected as the predetermined center point for the preparation chamber 100, and the center point N of the projection of the second collimator 201 on the wall where it is located is selected as the predetermined center point for the irradiation chamber 200. Of course, it is not limited to the above center point M and center point N, but other mutually corresponding points in the preparation chamber 100 and the irradiation chamber 200 may also be selected as the predetermined center points, as long as it is ensured that the preparation chamber 100 and the irradiation chamber 200 have the same reference base point.

The above is only a better embodiment of this application, and is not intended to limit this application, and any modifications, equivalent substitutions, etc. made within the spirit and principles of this application shall be included in the scope of protection of this application.

## Claims

1. A method of calibrating a laser positioning system, the method comprising:
providing a calibration device in each of a first chamber and a second chamber; wherein the calibration device comprises a calibration die, and a relative position of the calibration die of the first chamber to a predetermined center point of the first chamber is identical with a relative position of the calibration die of the second chamber to a predetermined center point of the second chamber; and
comparing a position of a first positioning mark on the calibration die of the first chamber and a position of a second positioning mark on the calibration die of the second chamber; wherein the first positioning mark is a positioning mark formed on the calibration die by a laser emitted by a first laser positioning system of the first chamber and the second positioning mark is a positioning mark formed on the calibration die by a laser emitted by a second laser positioning system of the second chamber.

2. The method of calibrating the laser positioning system according to claim 1, wherein the method further comprises:
adjusting the first laser positioning system and/or the second laser positioning system when there is a deviation between the position of the first positioning mark on the calibration die and/or the position of the second positioning mark on the standard die.

3. The method of calibrating the laser positioning system according to claim 1, wherein the calibration device further comprises a support frame;
providing the calibration device comprises: mounting the support frame, and providing the calibration die at a predetermined position on the support frame.

4. The method of calibrating the laser positioning system according to claim 3, wherein the support frame comprises a mounting bracket and a support plate mounted on the mounting bracket for supporting the calibration die; wherein
mounting the support frame comprises: mounting the mounting bracket in a predetermined position in the second chamber or the first chamber and adjusting angle and/or height of the support plate relative to the mounting bracket.

5. The method of calibrating the laser positioning system according to claim 4, wherein the support plate is provided with a first scale in a first direction and a second scale in a second direction perpendicular to the first direction; and
providing the calibration die at the predetermined position on the support frame comprises: placing the calibration die at a predetermined scale position on the support plate according to the first scale and the second scale.

6. The method of calibrating the laser positioning system according to claim 5, wherein in each of the first chamber and the second chamber, the support frame is mounted and adjusted to a horizontal level, and the calibration die located in each of the first chamber and the second chamber is placed in the predetermined scale position.

7. The method of calibrating the laser positioning system according to claim 6, wherein the calibration module is provided with a reference mark.

8. A calibration device for calibrating a laser positioning system, the calibration device being mounted in a first chamber having a first laser positioning system and/or a second chamber having a second laser positioning system;
the calibration device comprising a calibration die, wherein
a laser emitted by the first laser positioning system forms a first positioning mark on the calibration die when the calibration device is mounted in the first chamber, and a laser light emitted by the second laser positioning system forms a second positioning mark on the calibration die when the calibration device is mounted in the second chamber; consistency of the first laser positioning system and the second laser positioning system is determined based on a position of the first positioning mark on the calibration die and a position of the second positioning mark on the calibration die.

9. The calibration device according to claim 8, wherein the calibration device further comprises a support frame, the support frame is mounted in the first chamber or the second chamber, and the calibration die is provided on the support frame.

10. The calibration device according to claim 9, wherein the support frame comprises a mounting bracket and a support plate provided on the mounting bracket, the calibration die is selectively provided on the support plate;
wherein the support plate is adjustable in angle and/or height relative to the mounting bracket.

11. The calibration device according to claim 10, wherein the support plate is provided with a first scale in a first direction and a second scale in a second direction perpendicular to the first direction, and according to the first scale and the second scale, the calibration die is provided at a predetermined scale position on the support plate.

12. The calibration device according to claim 10, wherein the mounting bracket is provided with an adjusting structure located below the support plate, the support plate is provided with a positioning structure on a lower surface of the support plate which cooperates with the adjusting structure, and the angle and/or height of the support plate relative to the mounting bracket are adjusted by adjusting the adjusting structure.

13. The calibration device according to claim 11, wherein the calibration module is provided with a reference mark.

14. A calibration system, the calibration system comprising a first chamber provided with a first laser positioning system, a second chamber provided with a second laser positioning system, and a calibration device according to claim 7; wherein
the calibration device is provided in the first chamber and the second chamber; when a relative position between the calibration die of the first chamber and a predetermined center point of the first chamber and a relative position between the calibration die of the second chamber and a predetermined center point of the second chamber are identical, a position of a first positioning mark on the calibration die is compared with a second positioning mark of the second positioning mark on the calibration die to determine consistency of the first laser positioning system and the second laser positioning system; wherein the first positioning mark is a positioning mark formed on the calibration die of the first chamber by a laser emitted by the first laser positioning system and the second positioning mark is a positioning mark formed on the calibration die of the second chamber by a laser emitted by the second laser positioning system.

15. The calibration system according to claim 14, wherein each of the first chamber and the second chamber is provided with a collimator and a collimator mounting structure for mounting the collimator, and the support frame of the calibration device is mounted on the collimator mounting structure;
a predetermined center point of each of the first chamber and the second chamber corresponds to the collimator in position relationship, and is exactly consistent in spatial dimension.
